(19) Europäisches Patentamt
European Patent Office
Office européen des brevets

(11) **EP 2 591 309 B1**

(12) **EUROPEAN PATENT SPECIFICATION**

(45) Date of publication and mention
of the grant of the patent:
**26.11.2014 Bulletin 2014/48**

(51) Int Cl.:
***G01B 9/02*** *(2006.01)*        ***A61B 5/00*** *(2006.01)*
***A61B 3/12*** *(2006.01)*

(21) Application number: **11743885.3**

(22) Date of filing: **04.07.2011**

(86) International application number:
**PCT/JP2011/003803**

(87) International publication number:
**WO 2012/004970 (12.01.2012 Gazette 2012/02)**

(54) **OPTICAL TOMOGRAPHIC IMAGING APPARATUS**

OPTISCHES TOMOGRAFIEBILDGEBUNGSGERÄT

APPAREIL D'IMAGERIE TOMOGRAPHIQUE OPTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.07.2010 JP 2010156920**

(43) Date of publication of application:
**15.05.2013 Bulletin 2013/20**

(73) Proprietor: **Canon Kabushiki Kaisha
Tokyo 146-8501 (JP)**

(72) Inventors:
• **HIROSE, Futoshi
Tokyo 146-8501 (JP)**
• **SUGITA, Mitsuro
Tokyo 146-8501 (JP)**

(74) Representative: **Derham, Cassandra Virginie et al
Canon Europe Ltd
3 The Square
Stockley Park
Uxbridge
Middlesex
UB11 1ET (GB)**

(56) References cited:
**WO-A1-2010/074279**

• **ERICH GÖTZINGER ET AL: "Polarization
maintaining fiber based ultra-high resolution
spectral domain polarization sensitive optical
coherence tomography", OPTICS EXPRESS, vol.
17, no. 25, 7 December 2009 (2009-12-07), page
22704, XP55011242, ISSN: 1094-4087, DOI:
10.1364/OE.17.022704 cited in the application**

**Description**

[Technical Field]

The present invention relates to an optical tomographic imaging apparatus and an imaging method for the optical tomographic imaging apparatus. More particularly, the present invention relates to an optical tomographic imaging apparatus for capturing tomographic images used for ophthalmologic examinations, and to an imaging method for the optical tomographic imaging apparatus.

[Background Art]

Optical coherence tomography (OCT) using the multiple-wavelength optical interference is a method for obtaining a high-resolution tomographic image of a sample (particularly, the fundus). Hereinafter, an apparatus for capturing a tomographic image using OCT is referred to as an OCT apparatus.

In recent years, the Fourier Domain method enabling high-speed image capturing has been introduced to ophthalmologic OCT apparatuses. The Fourier Domain OCT apparatus can prevent blurring and missing of an image due to eye movements represented by the involuntary eye movement during visual-fixation. Japanese Unexamined Patent Application Publication No. 2008-508068 discusses a technique for detecting, by area sensors, a plurality of interference beams produced by return beams from the anterior ocular segment of the subject's eye irradiated with a plurality of measuring beams. Thus, the three-dimensional structure of the eyeball can be rendered at high speed by using a spline surface or a polygonal surface.

A functional OCT apparatus capable of imaging the polarization characteristics and movement of the fundus tissue is currently being researched. The functional OCT apparatus is different from an ordinary OCT apparatus which simply images the shape of the fundus tissue based on the boundary surface reflection and dispersion intensity. A polarization OCT apparatus, one type of the functional OCT apparatus, performs imaging by using polarization parameters (retardation and orientation) which are optical characteristics of the fundus tissue. As the result, it becomes possible to form a polarization OCT image using the polarization parameters and utilize the polarization OCT image for distinction and segmentation of the fundus tissue. The polarization OCT apparatus uses a circularly polarized beam or polarization-modulated beam as a measuring beam for observing a sample, and splits an interference beam into two orthogonal linearly polarized beams.

A technique for acquiring a high-resolution polarization OCT image by the polarization OCT apparatus having two spectroscopes and a wide-wavelength light source is discussed in "Polarization maintaining fiber based ultra-high resolution spectral domain polarization sensitive optical coherence tomography" Opt. Express 17, 22704 (2009). This technique renders a tomographic image in which the retinal pigment epithelium layer is distinguished from the fundus tissue by using acquired polarization parameters. It is known that depolarization for turning the polarized beam into a random beam takes place in the retinal pigment epithelium layer. The polarization OCT apparatus uses two diffraction gratings for two interference beams having different polarization directions (orthogonal linearly polarized beams).

From the above publication, which forms the closest prior art document, also an apparatus according to the preamble of claim 1 is known.

WO 2010/074279 describes an optical tomographic imaging apparatus in which multiple beams emitted from multiple light sources are split into a measuring beam group and a reference beam group, and in which a monitoring device for obtaining a monitoring image of the object which enable monitoring of an incident state represented by an incident position and an incident angle of the measuring beam group with respect to the object.

[Citation List]

[Patent Literature]

[PTL 1]

Japanese Unexamined Patent Application Publication No. 2008-508068 WO 2010/074279

[Non-Patent Literature]

[NPL 1]

"Polarization maintaining fiber based ultra-high resolution spectral domain polarization sensitive optical coherence tomography" Opt. Express 17, 22704 (2009)

[Summary of Invention]

[Technical Problem]

In this case, it is desirable that the OCT apparatus has functions of both high-speed image capturing and polarization OCT using a plurality of measuring beams.

If a polarization OCT image is acquired using all of the plurality of measuring beams, the OCT apparatus needs to be large-sized to detect return beams from the subject's eye because of the following reason: the required number of detection means such as line sensors, or the required number of detection areas for each measuring beam in the detection means is twice the number of measuring beams.

[Solution to Problem]

The present invention relates to a compact size optical tomographic imaging apparatus having functions of both high-speed image capturing and polarization OCT using a plurality of measuring beams, and to an imaging method for the optical tomographic imaging apparatus.

According to an aspect of the present invention, an optical tomographic imaging apparatus according to claim 1 is provided.

Further features and aspects of the present invention will become apparent from the following detailed description of exemplary embodiments with reference to the attached drawings.

[Brief Description of Drawings]

The accompanying drawings, which are incorporated in and constitute a part of the specification, illustrate exemplary embodiments, features, and aspects of the invention and, together with the description, serve to explain the principles of the invention.

Fig. 1 illustrates a configuration of an OCT apparatus according to a first exemplary embodiment of the present invention.

Fig. 2A illustrates a method of acquiring a tomographic image performed by the OCT apparatus according to the first exemplary embodiment of the present invention.

Fig. 2B illustrates a method of acquiring a tomographic image performed by the OCT apparatus according to the first exemplary embodiment of the present invention.

Fig. 2C illustrates a method of acquiring a tomographic image performed by the OCT apparatus according to the first exemplary embodiment of the present invention.

Fig. 3A illustrates a method of acquiring a tomographic image performed by the OCT apparatus according to the first exemplary embodiment of the present invention.

Fig. 3B illustrates a method of acquiring a tomographic image performed by the OCT apparatus according to the first exemplary embodiment of the present invention.

Fig. 3C illustrates a method of acquiring a tomographic image performed by the OCT apparatus according to the first exemplary embodiment of the present invention.

Fig. 3D illustrates a method of acquiring a tomographic image performed by the OCT apparatus according to the first exemplary embodiment of the present invention.

Fig. 4A illustrates an image capturing procedure performed by the OCT apparatus according to the first exemplary embodiment of the present invention.

Fig. 4B illustrates an image capturing procedure performed by the OCT apparatus according to the first exemplary embodiment of the present invention.

Fig. 5 illustrates an image capturing procedure performed by the OCT apparatus according to the first exemplary embodiment of the present invention.

[Description of Embodiments]

Various exemplary embodiments, features, and aspects of the invention will be described in detail below with reference to the drawings.

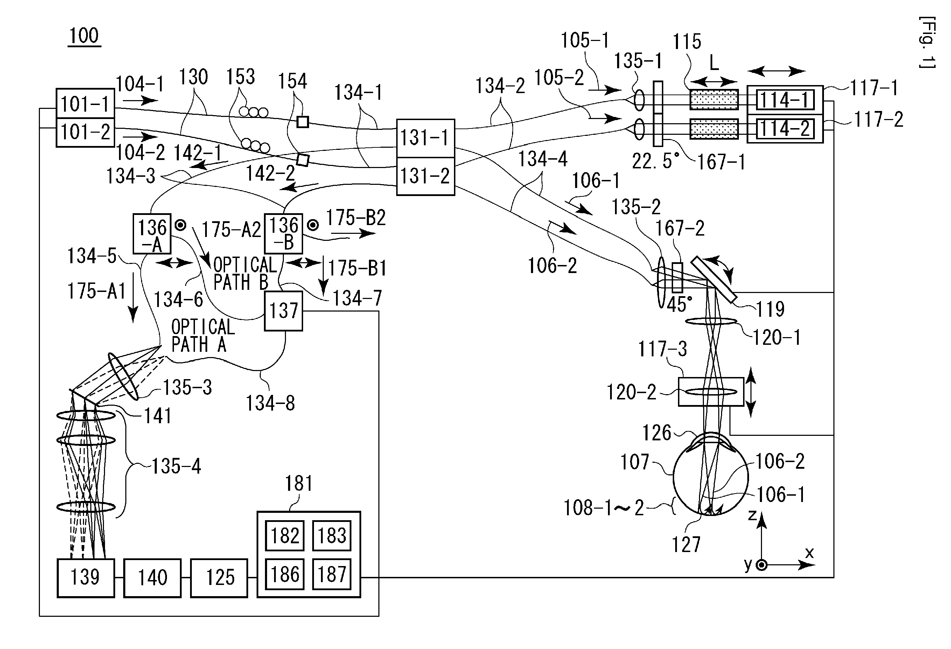

An imaging apparatus according to an exemplary embodiment of the present invention will be described below with reference to Fig. 1. Fig. 1 illustrates a configuration of an OCT apparatus (having functions of both ordinary OCT and polarization OCT) according to the present exemplary embodiment.

A return beam 108-1 comes from an inspection object 107 irradiated with a beam based on a first measuring beam 106-1 (a circularly polarized beam obtained after the first measuring beam 106-1 passes through a lambda/4 plate 167-2 disposed with a 45-degree (angle) inclination with respect to a measurement optical path). The return beam 108-1 is combined with a beam based on a reference beam 105-1 corresponding to the first measuring beam 106-1 (a linearly polarized beam obtained after the reference beam 105-1 passes through a lambda/4 plate 167-1 disposed with a 22.5-degree (angle) inclination with respect to a reference optical path) to form a first combined beam 142-1. The first combined beam 142-1 is split by a first splitting unit 136-A into a first beam 175-A1 and a second beam 175-A2 having different polarization directions.

A return beam 108-2 comes from the inspection object 107 irradiated with a beam based on a second measuring beam 106-2 (a circularly polarized beam obtained after the second measuring beam 106-2 passes through the lambda/4 plate 167-2 disposed with a 45-degree (angle) inclination with respect to the measurement optical path). The return beam 108-2 is combined with a beam based on a reference beam 105-2 corresponding to the second measuring beam 106-2 (a linearly polarized beam obtained after the reference beam 105-2 passes through the lambda/4 plate 167-1 disposed with a 22.5-degree (angle) inclination with respect to the reference optical path) to form a second combined beam 142-2. Either a third beam based on the second combined beam 142-2 or the second beam 175-A2 is selected by a selection unit (for example, an optical switch 137). The third beam is the second combined beam 142-2 itself or, when it is split by a second splitting unit 136-B into a third beam 175-B1 and a fourth beam 175-B2 having different polarization directions, the third beam 175-B1.

A case where the second beam 175-A2 is selected by the selection unit 137 will be described below. In this case, a first acquisition unit (for example, a computer 125) acquires a tomographic image (a polarization OCT image) indicating polarization information of the inspection object 107 based on the first beam 175-A1 and the second beam 175-A2 (this mode is referred to as a first mode).

A case where the third beam (the second combined beam 142-2 or the third beam 175-B1) is selected by the selection unit 137 will be described below. In this case, a second acquisition unit (for example, the computer 125) acquires tomographic images (a plurality of ordinary OCT images) indicating a plurality of pieces of intensity information of the inspection object 107 based on the first beam 175-A1 and the above-described third beam (this mode is referred to as a second mode). The first and second acquisition units may be formed of one common computer or two different computers.

In this way, the number of detection means or the number of the detection areas for each measuring beam in the detection means can be reduced to a number less than twice the number of measuring beams. Thus, it can be achieved a compact size optical tomographic imaging apparatus which has functions of both high-speed image capturing and polarization OCT using a plurality of measuring beams, and an imaging method for the optical tomographic imaging apparatus.

The above-described configuration means that the number of imaging areas for the polarization OCT is less than the number of imaging areas for the ordinary OCT having undergone high-speed image capturing by a plurality of measuring beams. More specifically, the range of the imaging areas for the polarization OCT is smaller than the total range of the imaging areas for the ordinary OCT. Thus, it is desirable to enable a user to determine the imaging areas for the polarization OCT as needed. Further thus, it is desirable to determine at least some imaging areas from an ordinary OCT image (wide-range image) captured at high-speed by the plurality of measuring beams, and then acquire a polarization OCT image in the determined imaging areas.

It is desirable to provide a spectroscopic unit (for example, a diffraction grating 141) for dispersing the first beam 175-A1 and the beam selected by the selection unit 137. In this case, it is desirable to provide an irradiation unit (for example, a lens 135-3) for irradiating an irradiation position on the spectroscopic unit 141 for the first beam 175-A1 with the beam selected by the selection unit 137. Thus, the optical tomographic imaging apparatus can be configured with one diffraction grating and therefore can be downsized than an apparatus including two diffraction gratings.

Further, it is desirable to provide a detection unit (for example, a line sensor 139) for detecting, in different detection areas, beams from the spectroscopic unit 141 respectively corresponding to the first beam 175-A1 and the beam selected by the selection unit 137. Thus, the plurality of beams can be detected by the common detection unit 139. Thus, a tomographic image indicating a plurality of pieces of intensity information of the inspection object or a tomographic image indicating polarization information can be acquired based on outputs from the detection areas of the detection unit 139 respectively corresponding to the first beam and the second beam.

Further, it is desirable to adjust the polarization direction of irradiation beams to a diffraction grating (for example, a light transmission type grating 141) respectively corresponding to the first beam 175-A1 and the second beam 175-A2 having different polarization directions. For example, it is desirable to adjust a relative angle formed between a light-emitting end of a first polarization maintaining fiber 134-5 and a light-emitting end of a second polarization maintaining fiber 134-8.

In this case, the spectral characteristics of the irradiation beams at the diffraction grating 141 (incidence beam polarization direction with respect to the diffraction grating 141) conform to each other. When the above-described adjustment is performed, it is desirable to irradiate the diffraction grating 141 with the first beam 175-A1 and the second beam 175-A2 with respective polarization directions aligned. In this case, it is desirable to acquire a tomographic image (also referred to as a polarization OCT image) indicating the polarization information of the inspection object 107 based on the beams from the diffraction grating 141 for dispersing the beams from the above-described adjustment unit respectively corresponding to the first beam 175-A1 and the second beam 175-A2 having different polarization directions.

Thus, the polarization direction of the incidence beams to the diffraction grating can be mutually aligned. Therefore, since the reduction in diffraction efficiency by the polarization dependency of the diffraction grating can be prevented, precision polarization parameters can be acquired to obtain a polarization OCT image with a simple configuration.

It is desirable to provide a display control unit for displaying a tomographic image of the inspection object on a display unit (display, etc.) based on a result of selection by the selection unit. It is desirable to provide a setting unit for setting either the first mode or the second mode. Further, the selection unit can be controlled based on a result of setting by the setting unit.

[Example 1]

In a first exemplary embodiment, an OCT apparatus to which the present invention is applied will be described below.

The first exemplary embodiment is formed as a multifunctional apparatus including polarization OCT for acquiring a polarization tomographic image and a multi-beam OCT including two measuring beams. The multifunctional apparatus can be switched between a polarization OCT mode and a multi-beam OCT mode.

In the polarization OCT mode, an inspection object is irradiated with a circularly polarized measuring beam from a light source, a return beam of the measuring beam radiated onto the inspection object is combined with a reference beam to produce an interference beam, the interference beam is split into two linearly polarized beams and measured, and a polarization OCT image of the inspection object is captured.

In the multi-beam OCT mode, for the purpose of high-speed image capturing and low-noise image capturing, two measuring beams are provided to enable simultaneously acquiring two tomographic images.

The OCT apparatus is characterized in acquiring detailed information of the inspection object. More specifically, the OCT apparatus first performs high-speed image capturing for a wide imaging range using two measuring beams in the multi-beam OCT mode, and then acquires polarization parameters for a target imaging range to be focused (for example, an affected area) in the polarization OCT mode.

Although the present exemplary embodiment is described centering on an OCT apparatus of a spectral domain type, it is obvious that the present invention is also applicable to an OCT apparatus of a time domain type or a swept source type.

<Overall Outline Configuration>

An overall configuration of the OCT apparatus according to the present exemplary embodiment will be described in detail below with reference to Fig. 1. As illustrated in Fig. 1, an OCT apparatus 100 according to the present exemplary embodiment totally configures a system as the Michelson interferometer and includes a polarization maintaining fiber capable of maintaining beam polarization state of a number of optical paths.

Referring to Fig. 1, an outgoing beam 104-1 emitted from a light source 101-1 is split into a reference beam 105-1 and a measuring beam 106-1 with an intensity ratio of 90:10 by an optical coupler 131-1, and an outgoing beam 104-2 emitted from a light source 101-2 is split into a reference beam 105-2 and a measuring beam 106-2 with the same intensity ratio by an optical coupler 131-2. The measuring beams 106-1 and 106-2 are led to the subject's eye 107 via polarization maintaining fibers 134-4, a XY scanner 119, and lenses 120-1 and 120-2.

The measuring beams 106-1 and 106-2 are reflected or scattered by the subject's eye 107 as an observation target to become the return beams 108-1 and 108-2, respectively. Then, the return beams 108-1 and 108-2 are combined with the reference beams 105-1 and 105-2 having passed through the reference optical path by the optical couplers 131-1 and 131-2, respectively.

After the return beams 108-1 and 108-2 are respectively combined with the reference beams 105-1 and 105-2, the resultant combined beam are dispersed for each wavelength by the light transmission type grating 141 and then input to the line camera 139 including one line sensor as a detection element. The line camera 139 converts optical intensity into a voltage for each position (wavelength), and the personal computer 125 uses the resultant signal to form a tomographic image of the subject's eye 107. Motor-driven stages 117-1 to 117-3, the XY scanner 119, the optical switch 137, and the light sources 101-1 and 101-2 are controlled and driven by the personal computer 125 via a driver unit 181.

<Light Sources>

Circumferences of the light sources 101-1 and 101-2 will be described below.

The light sources 101-1 and 101-2 are super luminescent diodes (SLDs) which are typical low-coherent light sources having a wavelength of 830 nm and a bandwidth of 50 nm. The bandwidth is an important parameter since it affects the resolution in the optical axis direction of the obtained tomographic image.

Although SLD is selected as the light source, the light source is not limited thereto, and any types of light sources can be employed as long as they can emit low-coherent beams, for example, an amplified spontaneous emission (ASE) light source. Further, in consideration of eye measurement, the wavelength of the near-infrared ray is suitable. Furthermore, since the wavelength affects the resolution in the horizontal direction of the obtained tomographic image, it is desirable to use an as short wavelength as possible. In the present exemplary embodiment, a wavelength of 830 nm is used. Depending on the measuring portion to be observed, other wavelengths may be selected.

Each of the outgoing beams 104-1 and 104-2 emitted from the light sources 101-1 and 101-2 passes through a single mode fiber 130, a polarization controller 153, a connector 154, and a polarization maintaining fiber 134-1. Then, the outgoing beams 104-1 and 104-2 are split into the reference beams 105-1 and 105-2 and the measuring beams 106-1 and 106-2 with an intensity ratio of 90: 10 by the optical couplers 131-1 and 131-2, respectively.

The OCT apparatus is configured such that the light sources 101-1 and 101-2 can be controlled by the personal computer 125 via a light source control driver 187 in the driver unit 181.

Although two outgoing beams from the two light sources are provided, a beam from one light source may be split into two to be used as two outgoing beams.

The polarization controller 153 has a function of adjusting the polarization state of emitted beams. In the present exemplary embodiment, the polarization state is adjusted to linearly polarized beams in the Y-axis direction (direction perpendicular to a paper surface of the drawing).

<Reference Optical Path>

Optical paths of the reference beams 105-1 and 105-2 will be described below.

The reference beams 105-1 and 105-2 split by the optical couplers 131-1 and 131-2 are led to lenses 135-1 via the polarization maintaining fibers 134-2 and then adjusted to become parallel beams having a 1 mm beam diameter. The reference beams 105-1 and 105-2 are linearly polarized in the Y-axis direction.

Then, the reference beams 105-1 and 105-2 respectively pass through a lambda/4 plate 167-1 (also referred to as a first change unit) and a dispersion compensation glass 115 to be lead to reference mirrors 114-1 and 114-2. Since the optical path lengths of the reference beams 105-1 and 105-2 are adjusted to approximately the same length as the optical path length of the measuring beams 106-1 and 106-2, respectively, the reference beams 105-1 and 105-2 can be interfered with the measuring beams 106-1 and 106-2, respectively. Then, the reference beams 105-1 and 105-2 are reflected by the mirrors 114-1 and 114-2 and then led again to the optical couplers 131-1 and 131-2, respectively.

The lambda/4 plate 167-1 is disposed such that its fast axis is inclined by 22.5 degrees (angle) with respect to the Y-axis direction. The reference beams 105-1 and 105-2 pass through the respective lambda/4 plates 167-1 twice to become linearly polarized beams inclined by 45 degrees (angle) with respect to the Y-axis direction. The linearly polarized beams inclined by 45 degrees (angle) contain a linearly polarized beam in the X-axis direction and a linearly polarized beam in the Y-axis direction.

The dispersion compensation glasses 115, which the reference beams 105-1 and 105-2 pass through, compensate the reference beams 105-1 and 105-2 for the dispersion occurring when the measuring beams 106-1 and 106-2 travel back and forth between the subject's eye 107 and the lenses 120-1 and 120-2. The diameter L of the eyeball is set to 24 mm which is an average value of Japanese.

The motor-driven stages 117-1 and 117-2 are movable in directions indicated by arrows, and capable of adjusting the optical path length for the reference beams 105-1 and 105-2. The motor-driven stages 117-1 and 117-2 can be controlled by the personal computer 125 via a motor-driven stage driver 183 in the driver unit 181.

<Measuring Optical Paths>

Optical paths of the measuring beams 106-1 and 106-2 will be described below.

The measuring beams 106-1 and 106-2 split by the optical couplers 131-1 and 131-2 are led to the a lens 135-2 via the polarization maintaining fibers 134-4 and then adjusted to become parallel beams having a 1 mm beam diameter. The measurement beams 106-1 and 106-2 pass through the lambda/4 plate 167-2 (also referred to as a second change unit), reflect off the XY scanner 119, pass through the lenses 120-1 and 120-2, and enters the subject's eye 107.

The lambda/4 plate 167-2 is disposed such that its fast axis is inclined by 45 degrees (angle) with respect to the Y-axis direction. The measurement beams 106-1 and 106-2 pass through the lambda/4 plate 167-2 to become circularly polarized beams before entering the subject's eye 107. The circularly polarized beams contain a linearly polarized beam in the X-axis direction and a linearly polarized beam in the Y-axis direction, having a 90-degree (angle) phase difference.

To simplify the description, the XY scanner 119 is described as one mirror, however it is actually formed of two mirrors (an X-scanning mirror and a Y-scanning mirror) disposed close to each other to raster-scan a retina 127 in a direction perpendicular to the optical axis. A center between the measuring beams 106-1 and 106-2 is adjusted to coincide with a rotational center of the two mirrors of the XY scanner 119. The XY scanner 119 is controlled by the personal computer 125 via an optical scanner driver 182 in the driver unit 181.

The lenses 120-1 and 120-2 form an optical system having a function of scanning the retina 127 with the measuring beams 106-1 and 106-2 using a vicinity of a cornea 126 as a fulcrum. Each of the lenses 120-1 and 120-2 has a focal length of 50 mm. Although the lens 120-2 is a spherical lens, it may be a cylindrical lens and a new lens may be added to the optical paths of the measuring beams 106-1 and 106-2 according to the optical aberration (refractive error) of the subject's eye 107. The use of a cylindrical lens is effective when the subject's eye 107 has astigmatism.

The motor-driven stage 117-3 is movable in a direction indicated by an arrow to adjust and control the position of the lens 120-2 accompanying the motor-driven stage 117-3. The motor-driven stage 117-3 is controlled by the personal computer 125 via the motor-driven stage driver 183 in the driver unit 181. The measuring beams 106-1 and 106-2 are condensed to a predetermined layer of the retina 127 of the subject's eye 107 by adjusting the position of the lens 120-2, so that the observation of the retina 127 can be performed. If the subject's eye 107 has a refractive error, the observation of the retina 127 can be performed by adjusting the position of the lens 120-2.

When the measuring beams 106-1 and 106-2 enter the subject's eye 107, the return beams 108-1 and 108-2 are produced by reflection and dispersion from the retina 127. The reference beams 105-1 and 105-2 are combined with the return beams 108-1 and 108-2 by the optical couplers 131-1 and 131-2 respectively, and then the resultant first combined beam 142-1 is split with an intensity ratio of 90:10.

<Measurement System>

A configuration of a measurement system in the OCT apparatus according to the present exemplary embodiment will be described below.

The combined beams 142-1 and 142-2 formed by the optical couplers 131-1 and 131-2 are led to polarization couplers 136-A and 136-B, respectively, via polarization maintaining fibers 134-3. The combined beam 142-1 is split into two split beams 175-A1 and 175-A2 which are linearly polarized beams orthogonal to each other. Likewise, the combined beam 142-2 is split into two split beams 175-B1 and 175-B2.

The split beams 175-A1 and 175-B1 are linearly polarized beams in the X-axis direction (direction in parallel with the paper surface of the drawing). The split beams 175-A2 and 175-B2 are linearly polarized beams in the Y-axis direction (direction perpendicular to the paper surface of the drawing). Further, the split beams 175-A2 and 175-B1 are input to the optical switch 137 via the polarization maintaining fibers 134-6 and 134-7, respectively.

The split beam 175-A1 is input to the lens 135-3 via the polarization maintaining fiber 134-5. The optical switch 137 selects either the split beam 175-A2 (optical path A) or the split beam 175-B1 (optical path B) and transmits the selected one to the lens 135-3 via the polarization maintaining fiber 134-8.

The optical switch 137 is controlled by the personal computer 125 via an optical switch driver 186 in the driver unit 181.

The OCT apparatus 100 is configured as the polarization OCT when the optical path A is selected or the multi-beam OCT when the optical path B is selected.

The optical switch 137 may be a mechanical beam switch for changing the optical path by mechanically moving an optical fiber or a micro-electro-mechanical system (MEMS) beam switch for changing the optical path by moving a micro mirror produced using the MEMS technology.

Either of the split beam 175-A1, the split beam 175-A2, or the split beam 175-B1 (referred to as a split beam 175-C) which are guided by the polarization maintaining fibers 134-5 and 134-8 is input to the lens 135-3. The split beams 175-A1 and 175-C reach the same position on the light transmission type grating 141 at different incident angles, and are dispersed for each wavelength by the light transmission type grating 141. The resultant beams are condensed by lenses 135-4 and then input to different positions of the line camera 139.

On the line camera 139, the light intensity of each of the split beams 175-A1 and 175-C is detected for respective positions (wavelengths). More specifically, a spectrum area interference fringe on the wavelength axis can be observed on the line camera 139. The detected light intensity is captured by the personal computer 125 using a frame grabber 140. The personal computer 125 performs data processing to form a tomographic image and displays it on a display screen (not illustrated).

The line camera 139 is provided with 2048 pixels and thus can obtain the light intensity for respective wavelengths (1024 divisions) of the split beams 175-A1 and 175-C.

<Tomographic Image Acquisition Method (Polarization OCT)>

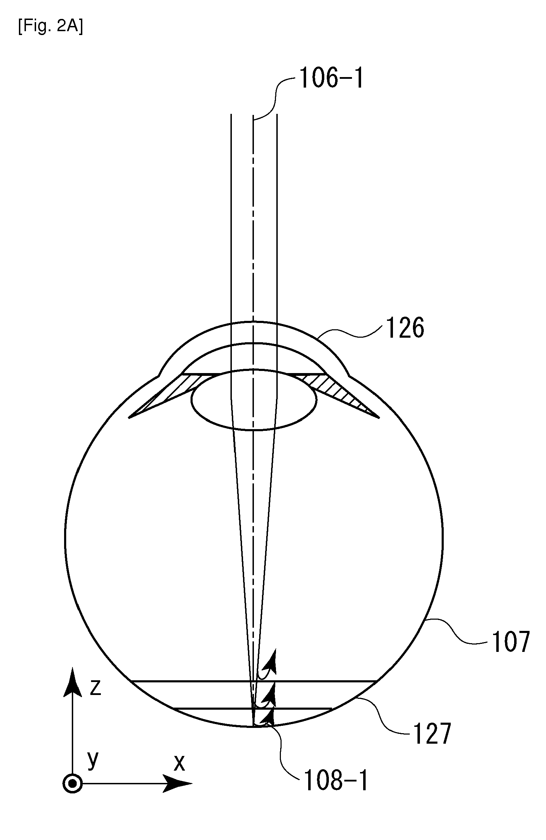

A method for acquiring a tomographic image by the OCT apparatus (the polarization OCT mode) will be described below with reference to Figs. 2A to 2C.

The OCT apparatus 100 controls the XY scanner 119 to acquire an interference fringe by the line camera 139, thus can acquire a tomographic image of the retina 127. In particular, a method for acquiring a polarization OCT image (a plane parallel to the optical axis, i.e., the XZ plane) forming a tomographic image using the polarization parameters, which is one of the optical characteristics of the fundus tissue, will be described below.

When a polarization OCT image is acquired, only the measuring beam 106-1 is used, thus the light source 101-1 is turned ON and the light source 101-2 is turned OFF using the personal computer 125 and the light source control driver 187. The optical path of the optical switch 137 is set to the optical path A by the personal computer 125 and the optical switch driver 186.

Fig. 2A is a schematic view illustrating the subject's eye 107 observed by the OCT apparatus 100. As illustrated in Fig. 2A, when the measuring beam 106-1 is input to the retina 127 via the cornea 126, the return beam 108-1 produced by reflection and dispersion at various positions reaches the line camera 139 with a time delay at each position.

When the reference beam optical path length is close to the measuring beam optical path length to some extent, an interference fringe that can be sampled for the pixel pitch is detected corresponding to the wavelength width of the light source 101, on the line camera 139. As described above, the line camera 139 acquires a spectrum area interference fringe on the wavelength axis in parallel for each polarization.

Then, the interference fringe, i.e. information on the wavelength axis, is converted into an interference fringe on the optical frequency axis in consideration of the characteristics of the line camera 139 and the light transmission type grating 141. Further, information about the depth direction can be acquired by applying the inverse Fourier transform to the converted interference fringe on the optical frequency axis.

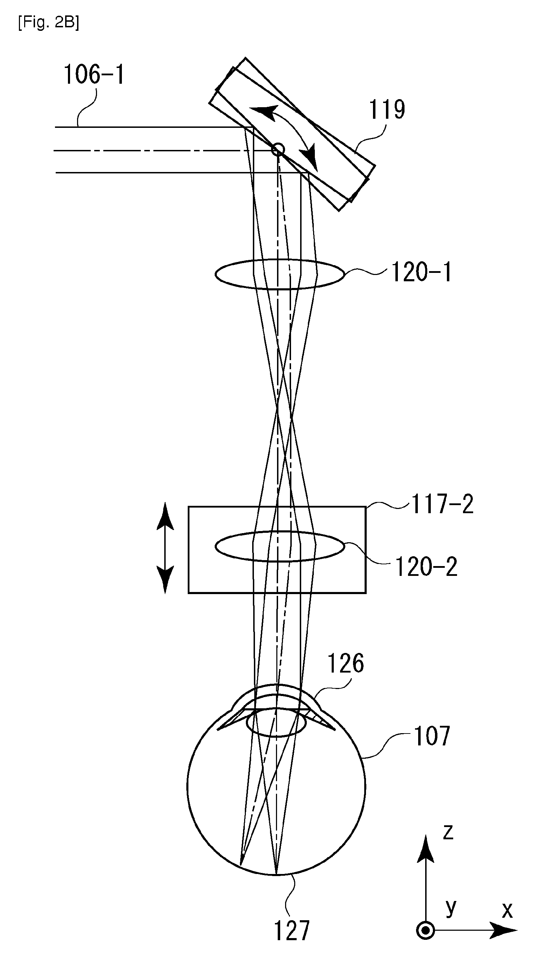

As illustrated in Fig. 2B, an interference fringe can be obtained at each X-axis position by detecting an interference fringe while driving the XY scanner 119. In other words, the information about the depth direction at each X-axis position can be obtained.

When interference signals with the split beams 175-A1 and 175-C have amplitudes $A_H$ and $A_V$, respectively, a reflectance R of an intensity OCT image, that is an ordinary OCT image, can be represented by formula (1). Non Patent Literature 1 is quoted in this case.

$$R \text{ is proportional to } A_H^2 + A_V^2 \quad \ldots\ldots \quad (1)$$

Retardation delta and orientation theta which are polarization parameters forming the polarization OCT image can be represented by formulas (2) and (3), respectively.

$$\text{delta} = \arctan[A_V/A_H] \quad \ldots\ldots \quad (2)$$

$$\text{theta} = (180 - \text{delta phi})/2 \quad \ldots\ldots \quad (3)$$

where phi is a phase of the split beams 175-A1 and 175-C, and delta phi is a phase difference between the amplitudes $A_H$ and $A_V$, and delta phi = $phi_H$ - $phi_V$ is satisfied.

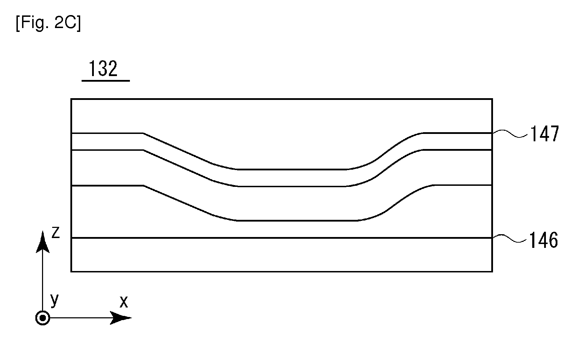

As a result, formula (1) gives a two-dimensional distribution of the intensity of the return beam 108-1 in the XZ plane, i.e., a tomographic image 132 (Fig. 2C). As described above, the tomographic image 132 is formed of the intensity of the return beam 108-1 in array form. For example, the intensity can be displayed according to a gray scale. Fig. 2C emphasizes only boundaries in the obtained tomographic image. The tomographic image 132 includes a pigment epithelium layer 146 and an inner limiting membrane 147.

Formulas (2) and (3) give a two-dimensional distribution of the polarization state of the return beam 108-1 on the XZ plane, i.e., a polarization OCT image. In particular, formula (2) gives a retardation image with which a relative phase delay between polarized beams is rendered. Further, formula (3) gives an orientation image which indicates change in polarization direction. Forming a polarization OCT image in this way makes it possible to render minute change in the fundus tissue such as a pigment epithelium cell which is said to cause depolarization, i.e. randomization of polarization, as well as change in the fibrous structure said to have large birefringence.

The amplitude AV of the interference signal 175-C is measured on the line camera 139 in response to light quantity loss at the optical switch 137. Therefore, it is preferable to correct the measured amplitude AV according to the specifications of the optical switch 137.

<Tomographic Image Acquisition Method (Multi-beam OCT)>

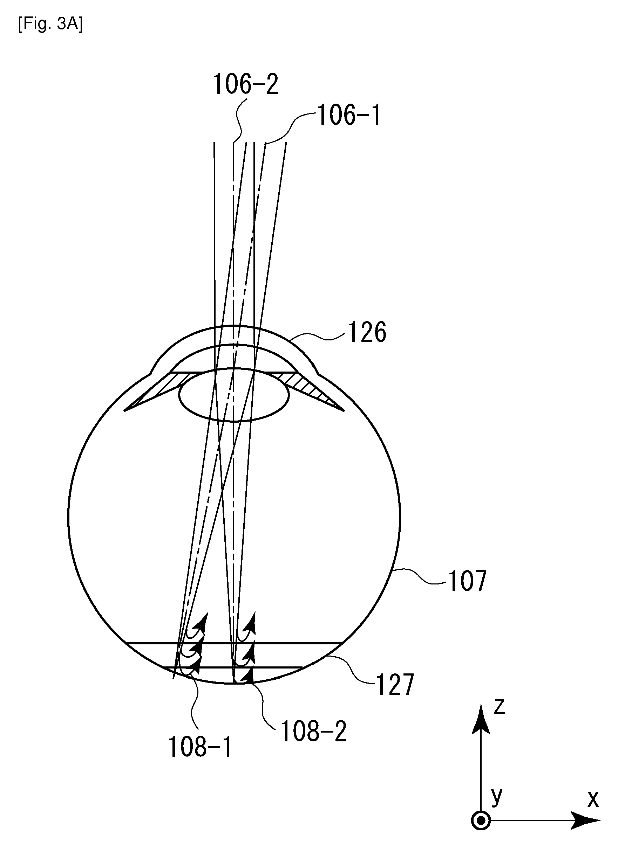

A method for acquiring a tomographic image by the OCT apparatus (the multi-beam OCT mode) with reference to Figs. 3A to 3D.

For the purpose of high-speed image capturing, the OCT apparatus 100 is configured to simultaneously acquire two tomographic images using two measuring beams 106-1 and 106-2.

When the two tomographic images are simultaneously acquired, both the measuring beams 106-1 and 106-2 are used, thus both the light sources 101-1 and 101-2 are turned ON using the personal computer 125 and the light source control driver 187. The optical path of the optical switch 137 is set to the optical path B by the personal computer 125 and the optical switch driver 186.

Fig. 3A is a schematic view illustrating the subject's eye 107 observed by the OCT apparatus 100. As illustrated in Fig. 3A, when the measuring beams 106-1 and 106-2 are input to the retina 127 via the cornea 126, the return beams 108-1 and 108-2 produced by reflection and dispersion at various positions reach the line camera 139 with a time delay at each position.

When the reference beam optical path length is close to the measuring beam optical path length to some extent, an interference fringe that can be sampled for the pixel pitch is detected corresponding to the wavelength width of the light source 101, on the line camera 139. As described above, a spectrum area interference fringe on the wavelength axis is acquired in parallel for each polarization on the line camera 139.

Then, the interference fringe, i.e. the information on the wavelength axis, is converted into an interference fringe on the optical frequency axis in consideration of the characteristics of the line camera 139 and the light transmission type grating 141. Further, the information about the depth direction can be acquired by applying the inverse Fourier transform to the converted interference fringe on the optical frequency axis.

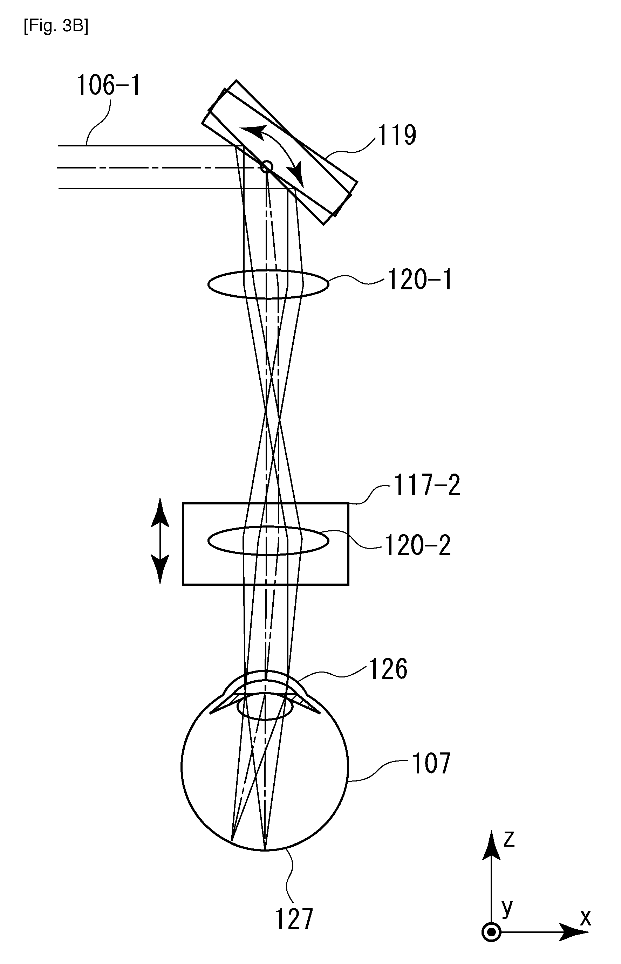

As illustrated in Fig. 3B, an interference fringe can be obtained at each X-axis position by detecting an interference fringe while driving the XY scanner 119. In other words, the information about the depth direction at each X-axis position can be obtained. For simplification of the description, only the measuring beam 106-1 is illustrated in Fig. 3B. As a result, a two-dimensional distribution of the intensity of the return beams 108-1 and 108-2 in the XZ plane, i.e., a plurality of tomographic images (not illustrated), can be obtained.

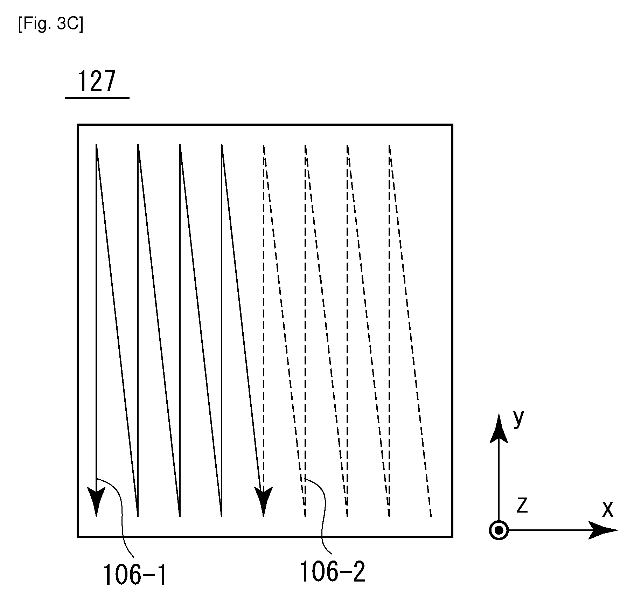

As illustrated in Fig. 3C, the OCT apparatus 100 controls the XY scanner 119 to use the measuring beams 106-1 (solid lines) and 106-2 (dashed lines) to raster-scan the retina 127, thus two tomographic images can be simultaneously acquired. Fig. 3C illustrates a case where the XY scanner 119 has the main scanning direction in the Y-axis direction and the subscanning direction in the X-axis direction, and as a result, a plurality of tomographic images in the YZ plane can be acquired. In this example, although the measuring beams 106-1 and 106-2 scan the retina 127 without overlapping with each other, they may scan it in an overlapped way, for example, for the purpose of correcting registration between tomographic images.

Since a tomographic image of an adjacent area obtained from the split beams 175-A1 and 175-C is used for registration correction, it is desirable that relevant optical paths have similar optical characteristics (transmissivity and polarization state). The polarization coupler 136-B is provided to correct the light quantity loss and achieve the same polarization state with respect to the polarization coupler 136-A.

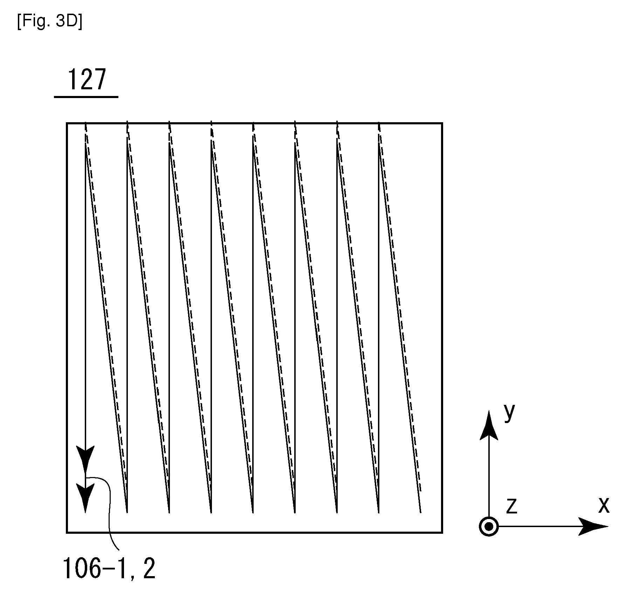

Further, as illustrated in Fig. 3D, the OCT apparatus 100 controls the XY scanner 119 to arrange the measuring beams 106-1 (solid lines) and 106-2 (dashed lines) in the Y-axis direction to raster-scan the retina 127, thus two tomographic images can be simultaneously acquired. In this case, the measuring beams 106-1 and 106-2 scan approximately the same position with a time difference. Therefore, interference signals acquired from the measuring beams 106-1 and 106-2 can be averaged to reduce noise in the acquired tomographic images. Further, a Doppler OCT apparatus can be configured which can calculate a blood flow velocity from a phase difference between the interference signals acquired from the measuring beams 106-1 and 106-2.

<Tomographic Image Capturing Procedure>

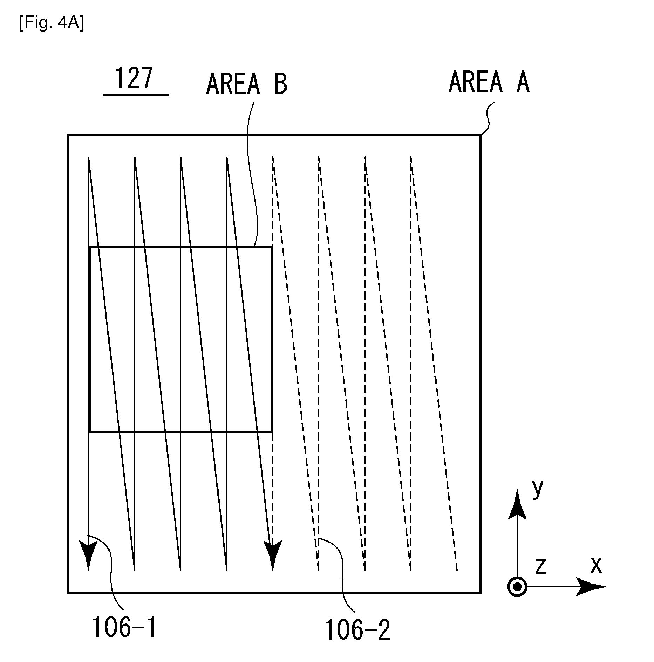

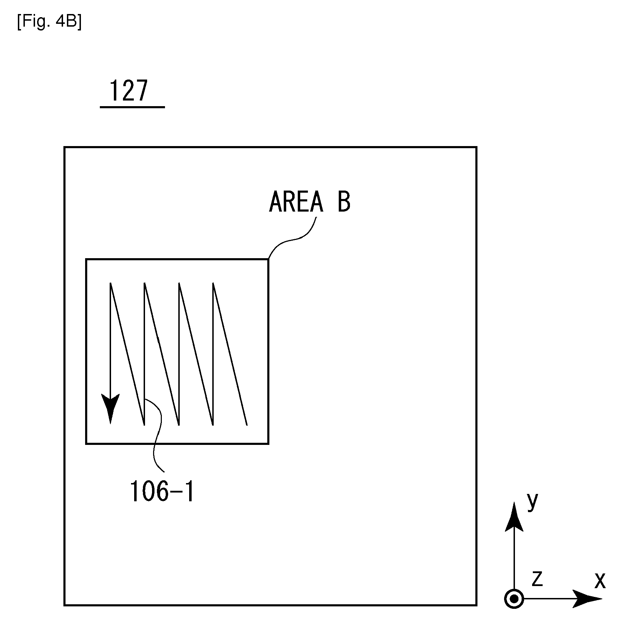

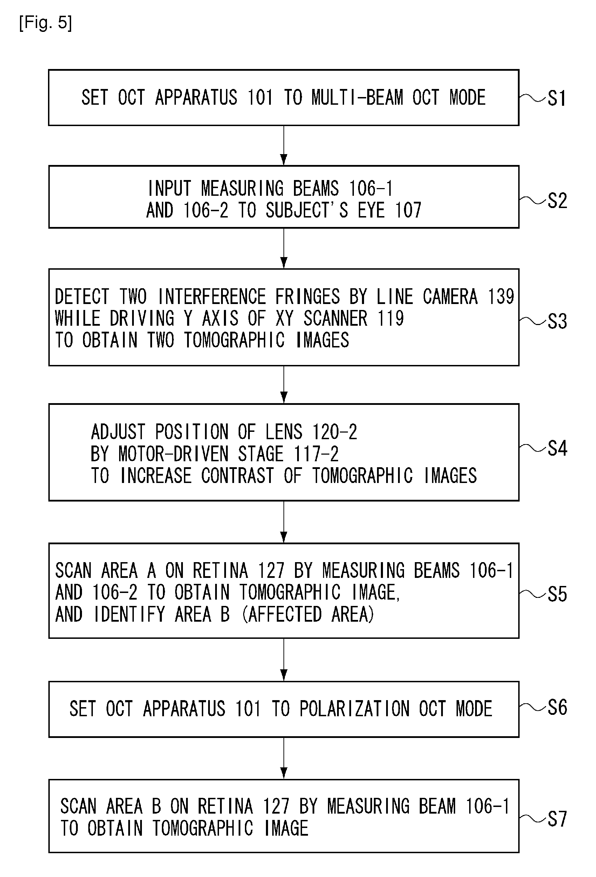

A procedure for capturing a tomographic image by the OCT apparatus will be described below with reference to Figs. 4A, 4B, and 5. The OCT apparatus first performs high-speed image capturing for a wide imaging range using two measuring beams in the multi-beam OCT mode to identify an affected area, and then performs more detailed observation of the identified affected area in the polarization OCT mode.

In the procedure for capturing a tomographic image, the following steps (1) to (7) may be performed in succession, for example. The procedure may return to a previous step as required. Further, the following steps may be automatically performed by a computer. Fig. 5 is a flow chart illustrating processing in each step of the method for capturing a tomographic image.

(1) In step S1, the OCT apparatus 100 sets the imaging mode to the multi-beam OCT mode. More specifically, the OCT apparatus turns ON the light sources 101-1 and 101-2 using the personal computer 125 and the light source control driver 187. Then, the OCT apparatus sets the optical path of the optical switch 137 to the optical path B using the personal computer 125 and the optical switch driver 186.

(2) In step S2, the OCT apparatus guides the subject's eye 107 to a predetermined position and, while making the subject's eye 107 gaze a fixation lamp (not illustrated), applies the measuring beams 106-1 and 106-2 to the subject's eye 107. In this processing, the position of the spherical mirror 120-2 is adjusted by the motor-driven stage 117-2 so that the measuring beams 106-1 and 106-2 are input to the subject's eye 107 in the form of parallel beams.

(3) In step S3, while driving the Y axis of the XY scanner 119, the OCT apparatus detects two interference fringes by the line camera 139 and obtains two tomographic images (not illustrated).

(4) In step S4, while performing the processing (3), the OCT apparatus adjusts the position of the lens 120-2 by the motor-driven stage 117-2 to increase the contrast of the tomographic images.

(5) In step S5, as illustrated in Fig. 4A, the area A on the retina 127 is scanned using the measuring beams 106-1 and 106-2 to obtain a tomographic image. In this processing, for the purpose of obtaining a wide range tomographic image, the measuring beams 106-1 and 106-2 basically scan different adjacent areas. In this case, it is assumed that an affected area candidate exists in the area B from the acquired tomographic image. Detailed descriptions on the extraction of an affected area candidate will be omitted here because there are various methods, for example, a method for analyzing each of a plurality of tomographic images forming the area A and then projecting the result to the fundus surface. With this image analysis method, a target area can be identified more accurately by using an analysis program dedicated for each individual disease.

(6) In step S6, the OCT apparatus 100 sets the imaging mode to the polarization OCT mode. More specifically, the OCT apparatus turns OFF the light source 101-2 using the personal computer 125 and the light source control driver 187. Then, the OCT apparatus sets the optical switch 137 to the optical path A using the personal computer 125 and the optical switch driver 186.

(7) In step S7, the OCT apparatus scans the area B on the retina 127 illustrated in Fig. 4B by the measuring beam 106-1 to obtain a tomographic image. In this processing, for the purpose of obtaining a detailed tomographic image of the target area, a polarization OCT image is acquired using the measuring beam 106-1.

Although the present exemplary embodiment is specifically described centering on two measuring beams in the multi-beam OCT mode, three or more measuring beams may be used to achieve the above-described configuration in the polarization OCT mode.

Although images are captured in the multi-beam OCT mode, and then in the polarization OCT mode, a mode setting switch for selecting the multi-beam OCT or the polarization OCT mode may be provided to enable image capturing in a selected mode.

As described above, selecting an arbitrary interference beam from a plurality of interference beams and performing detection enables one OCT apparatus to use both the polarization OCT mode and the multi-beam OCT mode, thus an OCT apparatus providing high space efficiency can be achieved.

Further, a polarization OCT apparatus can be configured to include means for splitting an interference beam for each polarized beam, and thus more detailed tomographic images of the inspection object can be acquired.

Further, using an optical switch as means for selecting an interference beam makes it possible to control the apparatus from outside using a personal computer or the like, and thus automated image capturing can be realized.

Further, a polarization OCT apparatus can be configured by splitting any arbitrary interference beam from a plurality of interference beams and performing detection, and thus more detailed tomographic images of the inspection object can be acquired.

Further, providing means for controlling emission and extinction of each of a plurality of beams makes it possible to control the apparatus from outside using a personal computer or the like, and thus automated image capturing can be realized.

Further, an OCT apparatus of spectral domain type can be configured by detecting an interference beam by a spectroscope having a diffraction grating, and thus high-speed image capturing can be achieved.

Further, two interference beams are input to the same position of a diffraction grating, and thus the space of the diffraction grating can be effectively utilized.

Further, a polarization OCT and a multi-beam OCT can be realized with a simple configuration by inputting two interference beams to one line camera and measuring their intensities.

The present invention can be achieved also by performing the following processing. More specifically, software (program) for implementing the functions of the above-described exemplary embodiment is supplied to a system or an apparatus via a network or various storage media, and a computer (or a central processing unit (CPU), a micro processing unit (MPU), etc.) of the system or the apparatus reads and executes the program.

**Claims**

1. An optical tomographic imaging apparatus (100) comprising:

   a first splitting unit (136-A) configured to split a first combined beam (142-1) into a first beam (174-A1) and a second beam (175-A2) having different polarization directions, the first combined beam being formed by combining a return beam (108-1) from an inspection object (107) irradiated with a first measuring beam (106-1) and a reference beam (105-1) corresponding to the first measuring beam; and
   a first acquisition unit (125) configured to acquire a tomographic image indicating polarization information of the inspection object (107) based on the first beam (175-A1) and the second beam (175-A2),

   **characterised in that** the optical tomographic imaging apparatus (100) further comprises:

   a selection unit (137) configured to select either a third beam (175-B1) or the second beam (175-A2), the third beam being based on a second combined beam (142-2) formed by combining a return beam (108-2) from the inspection object (107) irradiated with a second measuring beam (106-2) and a reference beam (105-2) corresponding to the second measuring beam, wherein the first acquisition unit (125) is configured to acquire the tomographic image based on the first beam (175-A1) and the second beam (175-A2) in a case wherein the second beam is selected by the selection unit (137); and
   a second acquisition unit (125) configured to acquire, in a case wherein the third beam (175-B1) is selected by the selection unit (137), a tomographic image indicating a plurality of pieces of intensity information of the inspection object (107) based on the first beam (175-A1) and the third beam (175-B1).

2. The optical tomographic imaging apparatus (100) according to claim 1, further comprising:

   a spectroscopic unit (141) configured to disperse the first beam and the beam selected by the selection unit (137); and
   an irradiation unit (134-5) configured to irradiate an irradiation position of the first beam (175-A1) on the spectroscopic unit (141) with the beam (175-A2,175-B1) selected by the selection unit.

3. The optical tomographic imaging apparatus (100) according to claim 2, further comprising a detection unit (139) configured to detect, in different areas, beams from the spectroscopic unit (141) respectively corresponding to the first beam (175-A1) and the beam (175-A2,175-B1) selected by the selection unit (137).

4. The optical tomographic imaging apparatus (100) according to any one of claims 1 to 3, further comprising:

   a first change unit (167-2) disposed in optical paths of the reference beams (105-1, 105-2) corresponding to the first (106-1) and second (106-2) measuring beams and configured to change a polarization direction of the reference beams; and
   a second change unit (167-2) disposed in optical paths of the first (106-1) and second (106-2) measuring beams and configured to change a polarization direction of the measuring beams.

5. The optical tomographic imaging apparatus (100) according to claim 4, wherein
   the first change unit (167-1) is configured to change the reference beams (105-1, 105-2) into linearly polarized beams; and
   the second change unit (167-2) is configured to change the measuring beams (106-1, 106-2) into circularly polarized beams.

6. The optical tomographic imaging apparatus (100) according to any one of claims 1 to 5, further comprising a second splitting unit (136-B) configured to split the second combined beam (142-2) into the third beam (175-B1) and a fourth beam (175-B2) having different polarization directions.

7. The optical tomographic imaging apparatus (100) according to claim 1, further comprising:

   an adjustment unit (134-5, 134-6, 134-8) configured to adjust, in a case wherein the second beam (175-A2) is selected by the selection unit (137), the polarization direction of irradiation beams to a diffraction grating respectively corresponding to the first and second beams so that spectral characteristics of the irradiation beams at the diffraction grating conform with each other;

the diffraction grating (141) configured to disperse the beams from the adjustment unit respectively corresponding to the first (175-A1) and second (175-A2) beams; and
a detection unit (139) configured to detect beams from the diffraction grating respectively corresponding to the first and second beams,
wherein the first acquisition unit (125) is configured to acquire a tomographic image indicating polarization information of the inspection object (107) based on outputs from the detection unit (139) respectively corresponding to the first and second beams.

8. The optical tomographic imaging apparatus (100) according to any one of claims 1 to 7, further comprising a display control unit configured to display a tomographic image of the inspection object on a display unit (132) based on a selection result of the selection unit (137).

9. The optical tomographic imaging apparatus (100) according to any one of claims 1 to 8, further comprising a setting unit configured to set either a first mode for selecting the second beam by the selection unit and a second mode for selecting the third beam by the selection unit,
wherein the selection unit is controlled based on a result of setting by the setting unit.

**Patentansprüche**

1. Optische Tomografiebildgebungsvorrichtung (100), umfassend:

eine erste Aufteileinrichtung (136-A), konfiguriert, um ein erstes kombiniertes Strahlenbündel (142-1) in ein erstes Strahlenbündel (174-A1) und ein zweites Strahlenbündel (174-A2) mit unterschiedlichen Polarisationsrichtungen aufzuteilen, wobei das erste kombinierte Strahlenbündel gebildet wird durch Kombinieren eines von einem mit einem ersten Messstrahlenbündel (106-1) bestrahlten Untersuchungsobjekt (107) zurückkehrenden Strahlenbündels (108-1) und eines dem ersten Messstrahlenbündel entsprechenden Referenzstrahlenbündels (105-1); und
eine erste Erfassungseinrichtung (125), konfiguriert, um ein Polarisationsinformation des Untersuchungsobjekts (107) angebendes Tomografiebild basierend auf dem ersten Strahlenbündel (175-A1) und dem zweiten Strahlenbündel (175-A2) zu erfassen,

**dadurch gekennzeichnet, dass** die optische Tomografiebildgebungsvorrichtung (100) weiter umfasst:

eine Auswähleinrichtung (137), konfiguriert, um entweder ein drittes Strahlenbündel (175-B1) oder das zweite Strahlenbündel (175-A2) auszuwählen, wobei das dritte Strahlenbündel auf einem zweiten kombinierten Strahlenbündel (142-2) basiert, das durch Kombinieren eines vom mit einem zweiten Messstrahlenbündel (106-2) bestrahlten Untersuchungsobjekt (107) zurückkehrenden Lichtstrahlenbündels (108-2) und eines dem zweiten Messstrahlenbündel entsprechenden Referenzstrahlenbündels (105-2) gebildet wird, wobei die erste Erfassungseinrichtung (125) konfiguriert ist, um das Tomografiebild basierend auf dem ersten Strahlenbündel (175-A1) und dem zweiten Strahlenbündel (175-A2) zu erfassen, falls das zweite Strahlenbündel durch die Auswähleinrichtung (137) ausgewählt wird; und
eine zweite Erfassungseinrichtung (125), konfiguriert, um, falls das dritte Strahlenbündel (175-B1) durch die Auswähleinrichtung (137) ausgewählt wird, basierend auf dem ersten Strahlenbündel (175-A1) und dem dritten Strahlenbündel (175-B1) ein mehrere Intensitätsinformationen des Untersuchungsobjekts (107) angebendes Tomografiebild zu erfassen.

2. Optische Tomografiebildgebungsvorrichtung (100) nach Anspruch 1, weiter umfassend:

eine spektroskopische Einrichtung (141), konfiguriert, um das erste Strahlenbündel und das durch die Auswähleinrichtung (137) ausgewählte Strahlenbündel zu dispergieren; und
eine Bestrahlungseinrichtung (134-5), konfiguriert, um eine Bestrahlungsposition des ersten Strahlenbündels (175-A1) auf der spektroskopischen Einrichtung (141) mit dem durch die Auswähleinrichtung ausgewählten Strahlenbündel (175-A2, 175-B1) zu bestrahlen.

3. Optische Tomografiebildgebungsvorrichtung (100) nach Anspruch 2, weiter umfassend eine Nachweiseinrichtung (139), konfiguriert, um in unterschiedlichen Bereichen jeweils dem ersten Strahlenbündel (175-A1) und dem durch die Auswähleinrichtung (137) ausgewählten Strahlenbündel (175-A2, 175-B1) entsprechende Strahlenbündel von der spektroskopischen Einrichtung (141) nachzuweisen.

4.  Optische Tomografiebildgebungsvorrichtung (100) nach einem der Ansprüche 1 bis 3, weiter umfassend:

    eine erste, in Strahlengängen der dem ersten (106-1) und dem zweiten (106-2) Messstrahlenbündel entsprechenden Referenzstrahlenbündel (105-1, 105-2) angeordnete Änderungseinrichtung (167-2), konfiguriert, um eine Polarisationsrichtung der Referenzstrahlenbündel zu ändern; und
    eine zweite, in Strahlengängen des ersten (106-1) und des zweiten (106-2) Messstrahlenbündels angeordnete Änderungseinrichtung (167-2), konfiguriert, um eine Polarisationsrichtung der Messstrahlenbündel zu ändern.

5.  Optische Tomografiebildgebungsvorrichtung (100) nach Anspruch 4, wobei
    die erste Änderungseinrichtung (167-1) konfiguriert ist, um die Referenzstrahlenbündel (105-1, 105-2) in linear polarisierte Strahlenbündel zu ändern; und
    die zweite Änderungseinrichtung (167-2) konfiguriert ist, um die Messstrahlenbündel (106-1, 106-2) in zirkular polarisierte Strahlenbündel zu ändern.

6.  Optische Tomografiebildgebungsvorrichtung (100) nach einem der Ansprüche 1 bis 5, weiter umfassend eine zweite Aufteileinrichtung (136-B), konfiguriert, um das zweite kombinierte Strahlenbündel (142-2) in das dritte Strahlenbündel (175-B1) und ein viertes Strahlenbündel (175-B2) mit unterschiedlichen Polarisationsrichtungen aufzuteilen.

7.  Optische Tomografiebildgebungsvorrichtung (100) nach Anspruch 1, weiter umfassend:

    eine Einstelleinrichtung (134-5, 134-6, 134-8), konfiguriert, um, falls das zweite Strahlenbündel (175-A2) durch die Auswähleinrichtung (137) ausgewählt wird, die Polarisationsrichtung jeweils dem ersten und dem zweiten Strahlenbündel entsprechender Bestrahlungsstrahlenbündel so bezüglich eines Beugungsgitter einzustellen, dass Spektralcharakteristiken der Bestrahlungsstrahlenbündel auf dem Beugungsgitter miteinander übereinstimmen;
    das Beugungsgitter (141), konfiguriert, um die jeweils dem ersten (175-A1) und dem zweiten (175-A2) Strahlenbündel entsprechenden Strahlenbündel von der Einstelleinrichtung zu dispergieren; und
    eine Nachweiseinrichtung (139), konfiguriert, um jeweils dem ersten und dem zweiten Strahlenbündel entsprechende Strahlenbündel vom Beugungsgitter nachzuweisen,
    wobei die erste Erfassungseinrichtung (125) konfiguriert ist, um ein Polarisationsinformation des Untersuchungsobjekts (107) angebendes Tomografiebild basierend auf jeweils dem ersten und dem zweiten Strahlenbündel entsprechenden Ausgaben von der Nachweiseinrichtung (139) zu erfassen.

8.  Optische Tomografiebildgebungsvorrichtung (100) nach einem der Ansprüche 1 bis 7, weiter umfassend eine Anzeigesteuereinrichtung, konfiguriert, um ein Tomografiebild des Untersuchungsobjekts auf einer Anzeigeeinrichtung (132) basierend auf einem Auswahlergebnis der Auswähleinrichtung (137) anzuzeigen.

9.  Optische Tomografiebildgebungsvorrichtung (100) nach einem der Ansprüche 1 bis 8, weiter umfassend eine Festlegungseinrichtung, konfiguriert, um entweder einen ersten Modus zum Auswählen des zweiten Strahlenbündels durch die Auswähleinrichtung oder einen zweiten Modus zum Auswählen des dritten Strahlenbündels durch die Auswähleinrichtung festzulegen,
    wobei die Auswähleinrichtung basierend auf einem Ergebnis der Festlegung durch die Festlegungseinrichtung gesteuert wird.

## Revendications

1.  Appareil optique (100) de formation d'image tomographique comprenant :

    une première unité (136-A) de séparation configurée pour séparer un premier faisceau combiné (142-1) en un premier faisceau (174-A1) et un second faisceau (175-A2) ayant des directions de polarisation différentes, le premier faisceau combiné étant formé par combinaison d'un faisceau (108-1) de retour provenant d'un objet examiné (107) irradié avec un premier faisceau (106-1) de mesure et un faisceau (105-1) de référence correspondant au premier faisceau de mesure ; et
    une première unité (125) d'acquisition configurée pour acquérir une image tomographique représentative d'une information de polarisation de l'objet examiné (107) basée sur le premier faisceau (175-A1) et le deuxième faisceau (175-A2),

l'appareil optique (100) de formation d'image tomographique étant **caractérisé en ce qu'**il comprend en outre :

une unité (137) de sélection configurée pour sélectionner soit un troisième faisceau (175-B1) soit le deuxième faisceau (175-A2), le troisième faisceau étant basé sur un second faisceau combiné (142-2) formé par combinaison d'un faisceau (108-2) de retour provenant de l'objet examiné (107) irradié avec un second faisceau (106-2) de mesure et d'un faisceau (105-2) de référence correspondant au second faisceau de mesure, la première unité (125) d'acquisition étant configurée pour acquérir l'image tomographique basée sur le premier faisceau (175-A1) et le deuxième faisceau (175-A2) dans un cas où le deuxième faisceau est sélectionné par l'unité (137) de sélection ; et

une seconde unité (125) d'acquisition configurée pour acquérir, dans un cas où le troisième faisceau (175-B1) est sélectionné par l'unité (137) de sélection, une image tomographique représentative d'une pluralité d'informations d'intensité de l'objet examiné (107) basée sur le premier faisceau (175-A1) et le troisième faisceau (175-B1).

2. Appareil optique (100) de formation d'image tomographique selon la revendication 1, comprenant en outre :

une unité spectroscopique (141) configurée pour disperser le premier faisceau et le faisceau sélectionné par l'unité (137) de sélection ; et

une unité (134-5) d'irradiation configurée pour irradier une position d'irradiation du premier faisceau (175-A1) sur l'unité spectroscopique (141) avec le faisceau (175-A2, 175-B1) sélectionné par l'unité de sélection.

3. Appareil optique (100) de formation d'image tomographique selon la revendication 2, comprenant en outre une unité (139) de détection configurée pour détecter, dans des zones différentes, des faisceaux provenant de l'unité spectroscopique (141) correspondant respectivement au premier faisceau (175-A1) et au faisceau (175-A2, 175-B1) sélectionné par l'unité (137) de sélection.

4. Appareil optique (100) de formation d'image tomographique selon l'une quelconque des revendications 1 à 3, comprenant en outre :

une première unité (167-2) de changement disposée dans les trajets optiques des faisceaux (105-1, 105-2) de référence correspondant aux premier (106-1) et second (106-2) faisceaux de mesure et configurée pour changer la direction de polarisation des faisceaux de référence ; et

une seconde unité (167-2) de changement disposée dans les trajets optiques des premier (106-1) et second (106-2) faisceaux de mesure pour changer la direction de polarisation des faisceaux de mesure.

5. Appareil optique (100) de formation d'image tomographique selon la revendication 4, dans lequel :

la première unité (167-1) de changement est configurée pour changer les faisceaux (105-1, 105-2) de référence en des faisceaux polarisés linéairement, et

la seconde unité (167-2) de changement est configurée pour changer les faisceaux (106-1, 106-2) de mesure en des faisceaux polarisés circulairement.

6. Appareil optique (100) de formation d'image tomographique selon l'une quelconque des revendications 1 à 5, comprenant en outre une seconde unité (136-B) de séparation configurée pour séparer le second faisceau combiné (142-2) en le troisième faisceau (175-B1) et un quatrième faisceau (175-B2) ayant des directions de polarisation différentes.

7. Appareil optique (100) de formation d'image tomographique selon la revendication 1, comprenant en outre :

une unité (134-5, 134-6, 134-8) d'ajustement configurée pour ajuster, dans un cas où le deuxième faisceau (175-A2) est sélectionné par l'unité (137) de sélection, la direction de polarisation des faisceaux d'irradiation sur un réseau de diffraction correspondant respectivement aux premier et second faisceaux de façon que les caractéristiques spectrales des faisceaux d'irradiation au niveau du réseau de diffraction soient conformes l'une à l'autre ;

le réseau de diffraction (141) configuré pour disperser les faisceaux provenant de l'unité d'ajustement correspondant respectivement aux premier (175-A1) et second (175-A2) faisceaux ; et

une unité (139) de détection configurée pour détecter des faisceaux provenant du réseau de diffraction correspondant respectivement aux premier et second faisceaux,
dans lequel la première unité (125) d'acquisition est configurée pour acquérir une image tomographique représentative d'une information de polarisation de l'objet examiné (107) basée sur des sorties de l'unité (139) de détection correspondant respectivement aux premier et second faisceaux.

8. Appareil optique (100) de formation d'image tomographique selon l'une quelconque des revendications 1 à 7, comprenant en outre une unité de commande d'affichage configurée pour afficher une image tomographique de l'objet examiné sur une unité (132) d'affichage en se basant sur un résultat de sélection de l'unité (137) de sélection.

9. Appareil optique (100) de formation d'image tomographique selon l'une quelconque des revendications 1 à 8, comprenant en outre une unité de paramétrage configurée pour paramétrer l'un ou l'autre d'un premier mode destiné à sélectionner le deuxième faisceau par l'unité de sélection et d'un second mode destiné à sélectionner le troisième faisceau par l'unité de sélection,
dans lequel l'unité de sélection est commandée en se basant sur le résultat de paramétrage par l'unité de paramétrage.

[Fig. 1]

EP 2 591 309 B1

[Fig. 2A]

[Fig. 2B]

[Fig. 2C]

[Fig. 3A]

[Fig. 3B]

[Fig. 3C]

127

106-1    106-2

[Fig. 3D]

<u>127</u>

106−1, 2

[Fig. 4A]

[Fig. 4B]

[Fig. 5]

SET OCT APPARATUS 101 TO MULTI-BEAM OCT MODE ∼S1

INPUT MEASURING BEAMS 106-1
AND 106-2 TO SUBJECT'S EYE 107 ∼S2

DETECT TWO INTERFERENCE FRINGES BY LINE CAMERA 139
WHILE DRIVING Y AXIS OF XY SCANNER 119
TO OBTAIN TWO TOMOGRAPHIC IMAGES ∼S3

ADJUST POSITION OF LENS 120-2
BY MOTOR-DRIVEN STAGE 117-2
TO INCREASE CONTRAST OF TOMOGRAPHIC IMAGES ∼S4

SCAN AREA A ON RETINA 127 BY MEASURING BEAMS 106-1
AND 106-2 TO OBTAIN TOMOGRAPHIC IMAGE,
AND IDENTIFY AREA B (AFFECTED AREA) ∼S5

SET OCT APPARATUS 101 TO POLARIZATION OCT MODE ∼S6

SCAN AREA B ON RETINA 127 BY MEASURING BEAM 106-1
TO OBTAIN TOMOGRAPHIC IMAGE ∼S7

**EP 2 591 309 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008508068 A

- WO 2010074279 A

**Non-patent literature cited in the description**

- Polarization maintaining fiber based ultra-high resolution spectral domain polarization sensitive optical coherence tomography. *Opt. Express,* 2009, vol. 17, 22704